# EUROPEAN PATENT APPLICATION

(11) **EP 1 806 150 A1**
(43) Date of publication of application: **11.07.2007**
(21) Application number: 06000168.2
(22) Date of filing: 05.01.2006
(51) Int. Cl.: A61K 47/10, A61K 38/14, A61P 31/04

(54) **Teicoplanin composition**

(71) Applicant: NEED PHARMA S.r.l., 20123 Milan (IT)
(72) Inventor: Vecchio, Carlo, 28010 Veruno (NO) (IT); Cavenaghi, Luigi, 20149 Milano (IT); Cozzi, Paolo, 20133 Milano (IT); Jabes, Daniela, 22070 Cassina Rizzardi (CO) (IT); Mosconi, Giorgio, 19087 Philadelphia (US)
(74) Representative: Trupiano, Federica

(57) **Abstract**

Object of the present invention is a formulation comprising Teicoplanin, a process for the preparation of said formulation and the use thereof as antibacterial agent.
Pharmaceutical compositions comprising the Teicoplanin formulation, and their use in the treatment of infections caused by aerobic and anaerobic Gram positive bacteria, are also an object of the present invention.

## Description

### OBJECT OF THE INVENTION

Object of the present invention is a formulation comprising Teicoplanin, a process for the preparation of said formulation and the use thereof as antibacterial agent.

Pharmaceutical compositions comprising the Teicoplanin formulation, and their use in the treatment of infections caused by aerobic and anaerobic Gram positive bacteria, are also an object of the present invention.

### STATE OF THE ART

Teicoplanin is a glycopeptide antibiotic fermentation product, produced by *Actinoplanes teichomyceticus,* and used as a drug since the end of the 1980s. Teicoplanin is a complex of three main components, denominated A1, A2 and A3. Factor A2, which is present in greatest quantities in the complex extracted from *Actinoplanes* fermentation broth, is the factor with most significant antibacterial activity. Teicoplanin factor A2 consists in turn of a complex of 5 components, all closely related one to each other. The structure of Teicoplanin has been comprehensively described and is reported, for example, in the Merck Index, 11th edition, 1989, page 1438, reference 9062, as well as also in the Journal of Hospital Infections, (1986) Vol.7, supplement A, pages 79-83.

Teicoplanin is an antibiotic which acts against the Gram positive bacteria responsible for serious infections such as endocarditis, dialysis associated peritonitis, and is also active against bacteria carrying resistance to other antibiotics. One of the situations where Teicoplanin is mostly used, is for treating infections with *Staphylococcus aureus,* a bacterial species responsible for septicaemia, poisoning and pneumonia, particularly widespread in hospital environments. *S. aureus* is the most aggressive of the staphylococcal bacteria and is the species which has always given the most problems, in that it developed the first penicillin-resistant strains as far back as 1942. Indeed, over the years, the bacterium has shown itself to be particularly capable of selecting novel resistant strains, producing mutations so rapidly as to leave the scientific world unprepared. Methicillin-resistant strains of *S. aureus* (MRSA, methicillin resistant *S*. *aureus)* are emerging with such increasing frequency that in some countries they represent over 50% of all the *S. aureus* strains isolated, Infections due to such pathogenic agents are typically recorded in hospital environments, where the antibiotic pressure exerted by the widespread use of such drugs has lead to the selection of resistant strains. Recently, the appearance of infections due to methicillin-resistant staphylococcal strains has also been observed in the community, and this observation has created a serious health scare.

In some recently described cases, *S. aureus* has shown resistance to Vancomycin, a glycopeptide antibiotic very similar to Teicoplanin, and also widely used in the treatment of Gram positive infections of the type described above. Teicoplanin has much more favourable pharmacokinetics with respect to Vancomycin, and may be administered in a single daily dose, both by intramuscular and intravenous injection, while it is not bioavailable if administered orally.

From the points mentioned above, the importance of Teicoplanin as antibiotic drug clearly appears.

However, said drug has certain drawbacks.

One of the main problems with Teicoplanin is represented by abundant foam production when the lyophilised product is reconstituted in solution. The procedure for the reconstitution of lyophilised Teicoplanin (for example a 200 mg ampoule) and administration, indicated in the instructions for use of the drug, mentions the following: Inject the WFI grade water slowly into the ampoule containing the active ingredient; gently roll the ampoule between the hands, until the powder is completely dissolved, trying to avoid the formation of any foam. An isotonic solution of approx. 66.7 mg/ml Teicoplanin, with a pH of 7.5 will be obtained. Any agitation, even slight, of the ampoule during reconstitution will result in the immediate production of abundant quantities of foam. Once the lyophilised product is dissolved in the ampoule with the solvent, the solution must be removed using a syringe in order to be transferred to the final container which will be used for the infusion, and the presence of foam greatly hinders this operation. Due to the foam that forms during the above described operation, the dose of active ingredient contained in the ampoule, may not be properly collected by the syringe in order to be administered to the patient, and hence a portion of the active ingredient may remain "trapped" by the foam produced, thus effectively impeding the administration of the correct dose of active ingredient, with potential consequences. Thus, the foam formation impedes the collection of constant quantities of drug for administration to the patient, with consequent high variability in the active ingredient dose. Said variability thus causes uncertainty in the administration of the drug, which must be absolutely avoided. In practice, there is never any true certainty of what effective dose has been administered, and this may lead to even serious consequences in the patient's pharmacological treatment, which may even be less effective. Indeed, it has been evaluated that the variability of dose caused by the formation of foam as described above, may even reach 50%.

Another problem associated with the formation of foam during the reconstitution of the solution from the lyophilised product is "regulatory" in nature, and is associated with determining the dose of Teicoplanin present in the ampoules for carrying out quality checks during the production of the drug. Indeed, when quality control checks are carried out on the various production batches of Teicoplanin, said checks are performed on the entire contents of the ampoule, dissolved as described above for the clinical administration of the product. Hence, again in this case, foam formation is a determining factor in contributing towards the variability of the measurement, with the immediate consequence that the quantity of Teicoplanin measured may oscillate dangerously, placing the quality of the batches produced at risk. Indeed, should a batch be characterised by quantities of active ingredient significantly lower than that envisaged, then the ampoule measuring-out system would be automatically corrected by increasing the quantity of Teicoplanin inserted into each ampoule. In reality, should the control measurement be "misrepresented" by the fact that the formation of foam has "subtracted" active ingredient from the solution used for checking, then there would be an adjustment of the quantity of active ingredient introduced, which might give rise to a batch that is actually overdosed with respect to the established amount. The end result might be that the administration to the patient might involve a greater dose than that envisaged, with potential, even negative, consequences. To obviate this drawback, the quantity of active ingredient affectively present in the ampoules is increased by approx. 10%, above all to ensure adequate treatment of the patients (overfilling), but even this work-around is poorly effective, as in any case it leads to high variability in the effective dosage of active ingredient.

### AIMS OF THE INVENTION

Hence, the aim of the present invention is that of providing a formulation comprising Teicoplanin which can be administered in reproducible, exact and constant doses.

An additional aim of the present invention is that of providing a Teicoplanin formulation which can be subjected to quality control, giving reliable and reproducible results.

Another aim of the invention is that of providing a formulation comprising Teicoplanin which does not produce foam when preparing the sample for administration or dosing.

Another aim of the present invention is that of providing a process for the preparation of a formulation comprising Teicoplanin,

Yet another aim of the invention is that of using said formulation comprising Teicoplanin for the treatment of infections caused by aerobic and anaerobic Gram positive bacteria, in addition to providing a pharmaceutical composition with antibiotic activity, to be used for the treatment of infections by Gram positive bacteria, and in particular *Staphylococcus aureus,* comprising said formulation.

### DESCRIPTION

These and other further aims and the relevant advantages, which will be further clarified by the following description, are achieved by a formulation comprising Teicoplanin and at least one anti-foaming compound selected from:

Sodium carboxymethylcellulose, sorbitol, mannitol, polyvinylpyrrolidone (PVP), Polyoxyethylene sorbitan monolaurate or monooleate, polysorbates or Tween 20 and 80, polyoxyethylene/polyoxypropylene/polyoxyethylene copolymer (Pluronic L-62), glycerol polyethylene glycol ricinoleate (Cremophor EL), silicone antifoam (Dimeticone), sorbitan monooleate or monolaurate (Span 20 and 80), Propylene glycol; polyethylene glycol 300 (PEG), Ethanol, dimethyl acetamide (DMA), glycerol, N-methyl-2-pyrrolidone (Pharmasolve), monothioglycerol.

Said antifoaming agent is advantageously used in quantities comprised of between 0.1 % by weight and 50 % by weight with respect to the quantity of Teicoplanin by weight, depending on the physico-chemical characteristics of the used agent. Again, according to the present invention, said antifoaming agent may be used in mixtures with at least one or more other antifoaming agent from among those indicated above. According to one preferred aspect of the present invention, said antifoaming agent is added to the liquid for the preparation of injectables.

According to the present invention, the formulation comprising Teicoplanin and at least one antifoaming compound, has the advantage of being able to be prepared for administration as an antibiotic without the formation of foam during resuspension of the lyophilisate, with the consequent possibility of withdrawing the entire suspension by syringe and thus being able to administer exactly the quantity of drug envisaged to the patient, without the introduction of any potentially harmful variables.

It is clear that, since the formulation is intended for administration to humans and pharmacological use, the antifoaming compound may certainly not be selected from conventional, known antifoaming compounds, given that said compounds are normally used in compositions of various types and intended for various uses, but certainly are not suggested for use in pharmaceutical compositions intended for administration to humans, particularly intramuscular or intravenous administration.

Indeed, administration in humans requires the use of completely compatible compounds, certainly far removed, from the physico-chemical viewpoint, from those normally used in the field of industrial antifoaming agents. Hence, the formulation according to the present invention is surprisingly compatible with the pharmacological use thereof, and may be administered to patients without any additional side effects with respect to those already envisaged for the active ingredient, Teicoplanin.

The reduced formation, or the absence of foam during solubilisation of the lyophilised product for the preparation of the Teicoplanin sample adapted to pharmacological administration or assaying, for example for quality control, brings significant advantages and allows maximum exploitation of the active ingredient in terms of efficacy, reproducibility of treatment and administered dose.

Particularly, in the case of quality control, by using the formulation according to the present invention, it will be possible to precisely assay the quantity of active ingredient in any given production batch and hence know exactly whether said active ingredient has been correctly measured, without having to continue with the addition of active ingredient in order to obviate potential losses during the solubilisation procedure.

The above described points will be further clarified through the following practical examples, given purely by way of non-limiting indication of the present invention.

### Example I

Teicoplanin - 200 mg
Sodium chloride - 24 mg
Silicone antifoam - 0.5- 2 mg

### Example II

Teicoplanin - 200 mg
Sodium chloride - 24 mg
Sodium carboxymethylcellulose - 5-20 mg

### Example III

Teicoplanin - 200 mg
Sodium chloride - 24 mg
Sorbital-45-100 mg

### Example IV

Teicoplanin - 200 mg
Sodium chloride - 24 mg
Ethanol - 10-40 mg

### Example V

Teicoplanin - 200 mg
Sodium chloride - 24 mg
Sorbitan monooleate (Span 80) - 1-10 mg

### Example VI

Teicoplanin - 200 mg
Sodium chloride - 24 mg
Polyoxyethylene sorbitan monooleate (Tween 80) - 2-10 mg

### Example VII

Teicoplanin - 200 mg
Sodium chloride - 24 mg
Polyoxyethylene/polyoxypropylene/polyoxyethylene copolymer (Pluronic L-62) - 1-10mg.

## Claims

1. A formulation comprising Teicoplanin and at least one antifoaming compound selected from: sodium carboxymethylcellulose, sorbitol, mannitol, polyvinylpyrrolidone (PVP), polysorbates 20 and 80 (Tween 20 and 80), polyoxyethylene-polyoxypropylene-polyoxyethylene copolymer (Pluronic L-62), glycerol polyethylene glycol ricinoleate (Cremophor EL), silicone antifoam (Dimeticone), sorbitan monooleate or monolaurate (Span 20 and 80), propylene glycol; polyethylene glycol 300 (PEG), ethanol, dimethyl acetamide (DMA), glycerol, N-methyl-2-pyrrolidone (Pharmasolve), monothioglycerol.

2. The formulation according to claim 1, **characterised in that** said antifoaming compound is present In quantities comprised of between 0.1 % by weight and 50 % by weight with respect to the quantity of Teicoplanin by weight.

3. A process for the preparation of the formulation according to claim 1, comprising a step involving the transfer of a liquid for the preparation of injectables into a suitable container containing the active ingredient, **characterised in that** said antifoaming agent is added to said liquid for the preparation of injectables.

4. Use of the formulation according to claim 1, for the treatment of infections caused by aerobic and/or anaerobic Gram positive bacteria.

5. A pharmaceutical composition comprising the formulation according to claim 1.

6. The pharmaceutical composition according to claim 5, **characterised in that** it comprises at least one of the following compounds:
Silicone antifoam, Sodium carboxymethylcellulose, Sorbitol, Ethanol, Sorbitan monooleate (Span 80), Polyoxyethylene sorbitan monooleate (Tween 80), Polyoxyethylene/polyoxypropylene/polyoxyethylene copolymer (Pluronic L-62).

7. Use of the pharmaceutical composition according to claim 5 as an antibiotic for the treatment of infections caused by aerobic and anaerobic Gram positive bacteria.
